(19) Europäisches Patentamt
European Patent Office
Office européen des brevets

(11) **EP 1 332 170 B1**

(12) **EUROPÄISCHE PATENTSCHRIFT**

(45) Veröffentlichungstag und Bekanntmachung des Hinweises auf die Patenterteilung:
**23.05.2007 Patentblatt 2007/21**

(21) Anmeldenummer: **01988742.1**

(22) Anmeldetag: **25.10.2001**

(51) Int Cl.:
*C08J 3/14* *(2006.01)*          *A61K 9/16* *(2006.01)*

(86) Internationale Anmeldenummer:
**PCT/EP2001/012354**

(87) Internationale Veröffentlichungsnummer:
**WO 2002/034816 (02.05.2002 Gazette 2002/18)**

(54) **VERFAHREN ZUR HERSTELLUNG VON MIKROSPHÄRISCHEN KRISTALLITEN AUS LINEAREN POLYSACCHARIDEN, ENTSPRECHENDE MIKROSPHÄRISCHE KRISTALLITE UND DEREN VERWENDUNG**

METHOD FOR PRODUCING MICROSPHERICAL CRYSTALLITES FROM LINEAR POLYSACCHARIDES, CORRESPONDING MICROSPHERICAL CRYSTALLITES AND THE USE THEREOF

PROCEDE POUR LA PRODUCTION DE CRITALLITES MICROSPHERIQUES A PARTIR DE POLYSACCHARIDES LINEAIRES, CRISTALLITES MICROSPHERIQUES CORRESPONDANTES ET LEUR UTILISATION

(84) Benannte Vertragsstaaten:
**BE CH DE FR GB LI NL**

(30) Priorität: **26.10.2000 DE 10053267**

(43) Veröffentlichungstag der Anmeldung:
**06.08.2003 Patentblatt 2003/32**

(73) Patentinhaber: **Südzucker AG Mannheim/ Ochsenfurt**
**68165 Mannheim (DE)**

(72) Erfinder:
• **HAUSMANNS, Stephan**
**65185 Wiesbaden (DE)**
• **KIY, Thomas**
**65929 Frankfurt am Main (DE)**
• **TOMKA, Ivan**
**CH-8057 Zürich (CH)**
• **MÜLLER, Rolf**
**CH-8055 Zürich (CH)**
• **MERTINS, Peter**
**35041 Marburg (DE)**

(74) Vertreter: **Luderschmidt, Schüler & Partner Patentanwälte**
**Industriepark Höchst**
**65926 Frankfurt (DE)**

(56) Entgegenhaltungen:
**WO-A-00/12590**          **DE-A- 19 737 481**

EP 1 332 170 B1

**Beschreibung**

Die vorliegende Erfindung betrifft ein neuartiges Verfahren zur Herstellung von mikrosphärischen Kristalliten, welche lineare Polysaccharide enthalten, diese mikrosphärischen Kristallite sowie deren Verwendung.

Verfahren zur Herstellung von Partikeln, insbesondere von Mikropartikeln aus Polymeren, wie z.B. Polysacchariden, für verschiedenste Anwendungen sind recht komplizierte Verfahren, die eine genaue Einhaltung unterschiedlicher Parameter voraussetzen. Insbesondere führen viele Verfahren auch nur zu geringen Ausbeuten und zu sehr breiten Partikelverteilungen. Zu nennen in diesem Zusammenhang sind vor allem Sprühtrocknung, Phasengrenzflächenkondensation und Emulsionsverfahren (z.B. WO-Verfahren = Wasser in Öl Emulsionen, WOW = Wasser in Öl in Wasser Emulsionen, Koazervation, Phasenseparation, Dispersion). Insbesondere Emulsionsverfahren, aber auch Sprühtrocknungen aus Zweiphasensystemen, erfordern ein sehr exaktes Vorgehen und in der überwiegenden Zahl der Fälle die Verwendung von Hilfsmitteln (Emulgatoren). Stabile Emulsionen sind oftmals nur mit hohem Aufwand und einer präzisen Kontrolle einer Vielzahl von Parametern (Temperatur, Rührgeschwindigkeit usw.) herzustellen, und die umfassende Abtrennung der Partikel bereitet Probleme. Die Ausbeute an Partikeln ist oft sehr niedrig, insbesondere ist die Einschlussrate von Wirksubstanzen ungenügend. Ein Aspekt, der im Fall teurer Pharmawirkstoffe die Anwendung einer Technologie verhindern kann.

Kugelförmige Mikropartikel, die neben Weinsäure enthaltenden Polykondensaten auch Ethylstärke oder sonstige Polysaccharide enthalten können, werden gemäß US-PS 5 391 696 einmal nach dem Verfahren der Sprühtrocknung erhalten, mit dem jedoch die Teilchengröße und besonders die Größenverteilung nur sehr schwer zu regeln ist. Eine weitere in dieser Patentschrift beschriebene Möglichkeit ist das Auflösen des Polymers in einem Lösungsmittel oder Lösungsmittelgemisch und das Eintropfen der Lösung in ein kaltes verflüssigtes Gas, z.B. flüssigen Stickstoff, wobei sich Mikropartikel bilden. Die Mikropartikel können dann in Wasser eingebracht werden, das gleichzeitig das Polymer ausfällt und das Lösungsmittel extrahiert. Dieses Verfahren ist umständlich, aufwendig und unökonomisch. Auch läßt die Gleichmäßigkeit der Partikeldimensionen zu wünschen übrig.

Die EP-B1-0 251 476 beschreibt die Herstellung von Mikropartikeln aus Polylactiden, in denen ein makromolekulares Polypeptid dispergiert ist. Auch hier ist eine intensive Kontrolle der verschiedensten Parameter erforderlich. Einheitliche sphärische Teilchen werden nicht erhalten.

Mikropartikel, die Wirkstoffe und Gase enthalten, werden in der WO 95/07 072 beschrieben. Die Herstellung erfolgt nach aufwendigen Emulsionsverfahren, die Größenverteilung der Partikel ist sehr uneinheitlich.

Yu Jiugao und Liu Jie berichten in starch/stärke 46(7), 252-5, (1994) über die Effekte der Suspensions-Vernetzungs-Reaktionsbedingungen auf die Größe von Stärke-Mikropartikel. Die Vernetzung findet in drei Stufen statt; das Medium ist eine Wasser-in-Öl Suspension, als Öl-Phase dient ein Erdnußöl/Toluol Gemisch. Vorgelatinisierte Stärke wird als wässrige Lösung, die noch Natriumhydroxid und Ethylendiamintetraessigsäure enthält, zugegeben. Ferner ist die Gegenwart eines oberflächenaktiven Mittels bzw. Stabilisators erforderlich.

Von Nachteil bei dem dort beschriebenen Verfahren ist, daß das Ergebnis von einer Vielzahl von Faktoren abhängig ist, nämlich von der Dichte, der Viskosität und den Konzentrationsverhältnissen sowohl der wäßrigen als auch der Öl-Phase, vom Stabilisator und von der Rührgeschwindigkeit, außerdem ist die Anwesenheit des Stabilisators nachteilig. Zudem ist es schwierig die Vielzahl der gegebenen Parameter zu kontrollieren, so daß die Reproduzierbarkeit nicht zufriedenstellend ist.

Mit makromolekularen Wirkstoffen beladene Teilchen aus wasserunlöslichen Polymeren wie Polymilchsäure oder Ethylcellulose werden entsprechend der Lehre der EP-B1-0 204 476 erhalten, indem man den partikulären Wirkstoff in einer acetonischen Lösung des Polymeren suspendiert und das Lösungsmittel bei Raumtemperatur abdampft. Die dabei entstehenden Teilchen zeigen noch nicht die gewünschten pharmakologischen Effekte, so daß eine Weiterverarbeitung zu sogenannten Pellets notwendig ist.

Die Druckschrift GB 2247242 offenbart ein Verfahren, Mikropartikel aus einem wasserlöslichem Material herzustellen, welches durch die Einwirkung des Enzyms CGTase (EC 2.4.1.19) und gegebenenfalls eines Stärke-entzweigenden Enzyms auf Cyclodextrine und/oder Stärke entsteht.

Die Druckschrift DE 197 37 481.6 der Anmelderin offenbart mikrosphärische Kristallite, die aus linearen, wasserunlöslichen Polysacchariden aufgebaut sind. Diese mikrosphärische Kristallite sind geeignet, die oben aufgezählten Nachteile zu überwinden. Insbesondere sind diese mikrosphärische Kristallite ebenmäßig aufgebaut, besitzen eine relativ große Einheitlichkeit und sind vielseitig verwendbar. In dieser Druckschrift sind außerdem Verfahren zur Herstellung diese mikrosphärische Kristallite beschrieben.

Das entsprechende Verfahren zur Herstellung dieser mikrosphärischen Kristalliten zeichnet sich u.a. dadurch aus, daß die linearen Polysaccharide in DMSO gelöst werden, und die mikrosphärische Kristallite durch Ausfällung in Wasser gebildet werden. Nachteilig hieran ist, daß die Produktionskosten durch Verwendung von DMSO relativ hoch liegen, daß DMSO-haltige Abfälle als Sonderabfälle zu entsorgen sind, und daß eine entsprechende Aufreinigung der mikrosphärischen Kristallite nötig ist, wenn diese beispielsweise im Pharma-, Kosmetik- oder dem Lebensmittelbereich eingesetzt werden sollen, da DMSO z.B. zur Herstellung von Kosmetika nicht verwendet werden darf (s. Deutsche Kosmetik-Verordnung Anlage 1, Nr. 338).

Es war daher Aufgabe der vorliegenden Erfindung, ein neuartiges Herstellungsverfahren zur Verfügung zu stellen, daß die vorstehend genannten Nachteile überwindet, und es ermöglicht, den in der DE 197 37 481.6 offenbarten mikrosphärischen Kristalliten ähnliche Kristallite ohne Verwendung von DMSO preisgünstiger und umweltfreundlicher herzustellen, wodurch diese für auch eine Verwendung in den Bereichen Pharma, Lebensmittel, Kosmetik besser geeignet sein würden.

Es wurde nun überraschend festgestellt, daß durch ein sehr einfaches Verfahren sehr einheitliche mikrosphärische Kristallite aus wasserunlöslichen linearen Polysacchariden in kristalliner Form in großen Mengen hergestellt werden können, die so mit ähnlichen kommerziell erhältlichen Polysacchariden, wie etwa Amylose oder Stärke, nicht erhalten werden, ohne daß zu ihrer Herstellung, wie in DE 197 37 481.6 beschrieben, DMSO verwendet werden muß.

Weiterhin wurde überraschend festgestellt, daß die so erhältlichen Mikrokristallite eine Kristallinität von mindestens 80% aufweisen.

Gegenstand der Erfindung ist daher ein Verfahren zur Herstellung von mikrosphärischen Kristalliten, welche ganz oder teilweise aus linearen Polysacchariden, insbesondere 1,4-α-D-Polyglucan bestehen, dadurch gekennzeichnet, daß man

(i) das (die) lineare(n) Polysaccharid(e) in Wasser bei 130-200°C und einem Druck p > 1 bar schmilzt, und durch
(ii) Fällen in einem Fällmittel bei 20-90°C die mikrosphärischen Kristallite herstellt.

Ein weiterer Gegenstand der Erfindung sind die mit dem obigen Verfahren erhältlichen mikrosphärischen Kristallite, deren Anteil an kritalliner Struktur bezogen auf die Gesamtstruktur (Kristallinitätsgrad) erfindungsgemäß mindestens 80%, bevorzugt mindestens 90%, besonders bevorzugt mindestens 95% und ganz besonders bevorzugt mehr als 98% ist.

Die Kristallinität der Kristallite kann beispielsweise mit der Röntgen-Strukturanalyse oder über die Dichte der Partikel auf an sich bekannte Weise bestimmt werden. Besonders bevorzugt wird die Kristallinität über die Röntgenweitwinkelstreuung bestimmt.

A- und B-Typ-Stärken unterscheiden sich durch ihren kristallinen Aufbau, wobei A-Typ-Stärken dichter gepackt sind und einen geringeren Wassergehalt aufweisen.

Erfindungsgemäß wird unter den Begriffen "Schmelzen" und "Schmelze" ein Vorgang bzw. ein Endpunkt eines Vorgangs gemeint, bei dem aus einer trüben Dispersion des Polyglucans in Wasser eine klare Lösung erhalten wird. Die Temperatur, bei der eine solche klare Lösung erhalten wird, hängt von dem Anteil des Wassers im Polyglucan/Wassergemisch ab.

Unter mikrosphärischen Kristalliten sind Kristallite, die annähernd Kugelform besitzen, zu verstehen. Bei Beschreibung einer Kugel durch von einem gemeinsamen Ursprung ausgehende, in den Raum gerichtete Achsen gleicher Länge, die den Radius der Kugel in allen Raumrichtungen definieren, ist für die mikrosphärischen Kristalliten eine Abweichung der Achsenlängen vom Idealzustand der Kugel von 1 % bis 40% möglich. Bevorzugt werden mikrosphärische Kristallite mit Abweichungen bis 25%, besonders bevorzugt bis 15% erhalten. Die Oberfläche der mikrosphärischen Kristallite kann makroskopisch mit der einer Himbeere verglichen werden, wobei die Tiefe der "Eindellungen" oder "Einschnitte" maximal 20% des mittleren Durchmessers der mikrosphärischen Kristallite betragen soll.

Lineare Polysaccharide im Sinne der vorliegenden Erfindung sind aus Monosacchariden als monomeren Bausteinen derart aufgebaut, daß die einzelnen Bausteine stets in der gleichen Art miteinander verknüpft sind. Jede so definierte Grundeinheit oder Baustein hat genau zwei Verknüpfungen, jeweils eine zu einem anderen Monomer. Davon ausgenommen sind lediglich die beiden Grundeinheiten, die den Anfang bzw. das Ende des Polysaccharids bilden. Diese haben nur eine Verknüpfung zu einem weiteren Monomer und bilden die Endgruppen des linearen Polysaccharids.

Besitzt die Grundeinheit drei oder mehr Verknüpfungen, wird von Verzweigung gesprochen. Dabei ergibt sich aus der Anzahl der Hydroxylgruppen pro 100 Grundeinheiten, die nicht am Aufbau des linearen Polymerrückgrats beteiligt sind und die Verzweigungen ausbilden, der sogenannte Verzweigungsgrad.

Erfindungsgemäß weisen die linearen wasserunlöslichen Polysaccharide einen Verzweigungsgrad von maximal 2,5 % auf, d.h. 25 Verzweigungen auf 1000 Monomere.

Bevorzugt sind Polysaccharide deren Verzweigungsgrad in 6-Position kleiner 1 %, vorzugsweise maximal 0,5 %, und in den anderen Positionen, z. B. in 2- bzw. 3-Position, vorzugsweise jeweils maximal 2 % und insbesondere maximal 1 % ist.

Besonders bevorzugt sind auch Polysaccharide, deren Verzweigungsgrad in 6-Position kleiner als 0,5 % ist.

Für die Erfindung sind insbesondere Polysaccharide geeignet, die keine Verzweigungen aufweisen.

In Ausnahmefällen kann deren Verzweigungsgrad so minimal sein, daß er mit herkömmlichen Methoden nicht mehr nachweisbar ist.

Unter dem Begriff "wasserunlösliche Polysaccharide" werden für die vorliegende Erfindung Verbindungen verstanden, die nach der Definition des Deutschen Arzneimittelbuches (DAB = Deutsches Arzneimittelbuch, Wissenschaftliche Verlagsgesellschaft mbH, Stuttgart, Govi-Verlag GmbH, Frankfurt, 9. Auflage, 1987) entsprechend den Klassen 4 bis 7 unter die Kategorien "wenig löslich", "schwer lösliche", "sehr schwer lösliche" bzw. "praktisch unlösliche" Verbindungen fallen.

Erfindungsgemäß bevorzugte Polysaccharide lassen sich daher der Klasse 4 des DAB zuordnen, d.h. daß eine gesättigte Lösung des Polysaccharids bei Raumtemperatur und Normaldruck etwa 30 bis 100 Volumenteile Lösungsmittel, d.h. Wasser, pro Massenteil Substanz umfaßt (1g Substanz auf 30-100ml Wasser). Erfindungsgemäß mehr bevorzugte Polysaccharide lassen sich der Klasse 5 des DAB zuordnen, d.h. daß eine gesättigte Lösung des Polysaccharids bei Raumtemperatur und Normaldruck etwa 100 bis 1000 Volumenteile Lösungsmittel, d.h. Wasser, pro Massenteil Substanz umfaßt (1g Substanz auf 100-1000ml Wasser). Erfindungsgemäß noch mehr bevorzugte Polysaccharide lassen sich der Klasse 6 des DAB zuordnen, d.h. daß eine gesättigte Lösung des Polysaccharids bei Raumtemperatur und Normaldruck etwa 1000 bis 10000 Volumenteile Lösungsmittel, d.h. Wasser, pro Massenteil Substanz umfaßt (1g Substanz auf 1000-10000ml Wasser). Erfindungsgemäß am meisten bevorzugte Polysaccharide lassen sich der Klasse 7 des DAB zuordnen, d.h. daß eine gesättigte Lösung des Polysaccharids bei Raumtemperatur und Normaldruck etwa 10000 bis 100000 Volumenteile Lösungsmittel, d.h. Wasser, pro Massenteil Substanz umfaßt (1g Substanz auf 10000-100000ml Wasser).

Der durchschnittliche Polymerisationsgrad (DP) der erfindungsgemäßen Polysaccharide beträgt 20 - 400, bevorzugt 30 - 150, besonders bevorzugt 44 - 100, und ganz besonders bevorzugt 50 - 70. Im Falle des besonders bevorzugten poly-$\alpha$-1,4-D-Glucans läßt sich der DP durch einfache Division des $M_n$, d.h. des Zahlenmittels des Molekulargewichts, durch das Molekulargewicht der einzelnen Untereinheit (etwa 162), ermitteln.

Im Rahmen der Erfindung werden bevorzugt lineare, wasserunlösliche Polysaccharide, welche in einem biotechnischen, insbesondere in einem biokatalytischem bzw. biotransformatorischem, oder einem fermentativen Prozeß hergestellt wurden.

Lineare Polysaccharide hergestellt durch Biokatalyse (auch: Biotransformation) im Rahmen dieser Erfindung bedeutet, daß das lineare Polysaccharid durch katalytische Reaktion von monomeren Grundbausteinen wie oligomeren Sacchariden, z.B. von Mono- und/oder Disacchariden, hergestellt wird, indem ein sogenannter Biokatalysator, üblicherweise ein Enzym, unter geeigneten Bedingungen verwendet wird.

Linearer Polysaccharide aus Fermentationen sind im Sprachgebrauch der Erfindung lineare Polysaccharide, die durch fermentative Prozesse unter der Verwendung in der Natur vorkommende Organismen, wie Pilzen, Algen oder Bakterien oder unter der Verwendung von in der Natur nicht vorkommender Organismen, aber unter Zuhilfenahme von gentechnischen Methoden allgemeiner Definition modifizierten natürlichen Organismen, wie Pilzen, Algen oder Bakterien gewonnen werden oder unter Einschaltung und Mithilfe von fermentativen Prozessen gewonnen werden können.

Lineare Polymere gemäß der vorliegenden Erfindung können neben dem bevorzugten 1,4-$\alpha$-D-Polyglucan auch weitere Polyglucane oder andere lineare Polysaccharide wie etwa Pektine, Mannane oder Polyfructane sein.

Darüber hinaus können lineare Polymere zur Herstellung der in der vorliegenden Erfindung beschriebenen mikrosphärischen Kristallite auch aus der Reaktion weiterer nicht linearer Polysaccharide dadurch gewonnen werden, daß nicht lineare Polysaccharide, die Verzweigungen enthalten, derart mit einem Enzym behandelt werden, daß es zur Spaltung der Verzweigungen kommt, so daß nach ihrer Abtrennung lineare Polysaccharide vorliegen. Bei diesen Enzymen kann es sich beispielsweise um Amylasen, iso-Amylasen, Gluconohydrolasen oder Pullulanasen handeln.

In einer besonders vorteilhaften Ausführungsform der Erfindung bestehen die mikrosphärischen Kristallite ganz oder teilweise aus 1,4-$\alpha$-D-Polyglucan. Bevorzugt wird das 1,4-$\alpha$-D-Polyglucan mittels eines biokatalytischen (biotransformatorischen) Prozesses mit Hilfe von Polysaccharidsynthasen, Stärkesynthasen, Glykosyltransferasen, $\alpha$-t,4-Glucantransferasen, Glycogensynthasen, Amylosucrasen oder Phosphorylasen hergestellt.

In einer weiteren vorteilhaften Ausführungsform werden die linearen, wasserunlöslichen Polysaccharide, insbesondere das 1,4-$\alpha$-D-Polyglucan, durch enzymatische Behandlung von verzweigten oder hochverzweigten Polysacchariden hergestellt.

Erfindungsgemäß wird ausschließlich Wasser für das Schmelzen der linearen Polysaccharide eingesetzt.

Als Fällmittel ist Wasser bevorzugt; der Prozeß kann durch die Verwendung anderer Lösungsmittel, die Wasser ganz oder teilweise ersetzen können wie z.B. Dichlormethan, beeinflußt werden, wobei u.a. die Dauer des Fällprozesses und die Struktur der Oberfläche der Partikel gesteuert werden können.

Auch Gemische von Wasser mit Alkoholen, z.B. Methanol, Ethanol, Isopropanol, sind als Fällmittel dazu geeignet, die Prozeßparameter sowie die Eigenschaften der Partikel zu beeinflussen.

Die Temperatur während des Fällprozesses liegt vorzugsweise bei etwa 20°C, es können jedoch auch höhere oder tiefere Temperaturen verwendet werden.

Es hat sich gezeigt, daß es besonders günstig ist, die verwendeten Polysaccharide, besonders das 1,4-$\alpha$-D-Polyglucan, vor Züchtung der mikrosphärischen Kristallite durch wiederholtes Schmelzen und Fällen in Wasser zu reinigen. Dieser wiederholte Auflösungs- und Fällprozeß vorgängig zur Züchtung der mikrosphärischen Kristallite führt zu einer Polysaccharidfraktion, in der weder niedermolekulare Saccharide noch Proteine oder Nukleinsäuren nachgewiesen werden können.

Eine bevorzugte Ausführungsform der vorliegenden Erfindung ist daher ein Verfahren, bei dem man das wasserunlösliche lineare Polysaccharid, bevorzugt das 1,4-$\alpha$-D-Polyglucan, vorgängig zur Bildung von mikrosphärischen Kristalliten mehrmals im Wasser schmilzt und ausfällt.

Das Züchten der mikrosphärischen Kristallite geschieht erfindungsgemäß durch Schmelzen der wie oben geschildert gereinigten Polysaccharide, bevorzugt des gereinigten 1,4-$\alpha$-D-Polyglucans, in Wasser bei Eigendruck und einer Temperatur von 130-200°C und nachfolgendem Fällen bei $\leq$ 90°C.

Weiter können die Prozeßführung sowie die Eigenschaften der Partikel, wie Größe, Verteilung der Größe und die Beschaffenheit - glatt oder rauh - der Oberfläche durch Zusatz weiterer Hilfsmitteln für die Kristallzüchtung beeinflußt werden.

Geeignete Hilfsmittel, die neben dem heiß- oder kaltwasserlöslichen Stärke zum Einsatz kommen können, sind z.B. oberflächenaktive Stoffe wie Natriumdodecylsulfat, N-Methylgluconamid, Polysorbate (z.B. Tween (eingetragene Marke)), Alkylpolyglycolether, Ethylenoxid-Propylenoxid-Blockpolymere (z.B. Pluronic (eingetragene Marke)), Alkylpolyglycolethersulfate, generell Alkylsulfate und Fettsäureglycolester, Zucker wie z.B. Fructose, Saccharose, Glucose und wasserlösliche Cellulosederivate.

Die oberflächenaktiven Stoffe können anionischer, kationischer oder nichtionischer Natur sein.

Durch Zusatz von wasserlöslichen Cellulosederivaten können besonders regelmäßige, d.h. glatte, Oberflächen erhalten werden. Prinzipiell kann jedes wasserlösliche Cellulosederivat verwendet werden, sofern es als Fällungshilfsmittel geeignet ist. Es kann sich hierbei um chemisch modifizierte Cellulosen jedweder Art handeln. Beispiele sind Celluloseester und Celluloseether und deren Mischformen. Konkrete Vertreter sind z.B.

Hydroxypropylmethylcellulosen, Hydroxyethylcellulosen, Carboxymethylcellulosen, Celluloseacetate, Cellulosebutyrate, Cellulosepropionate, Cel lu loseacetobutyrate, Cellu loseacetopropionate, Cellulosenitrate, Ethylcellulosen, Benzylcellulosen, Methylcellulosen etc.

Es können auch Mischungen von verschiedenen wasserlöslichen Cellulosederivaten eingesetzt werden.

Die Partikel können mittlere Durchmesser (Zahlenmittelwert) aufweisen wie 1 nm bis 100 $\mu$m, bevorzugt 50 nm bis 10 $\mu$m, besonders bevorzugt 100 nm bis 5 $\mu$m.

Die Partikel zeigen ein Verhältnis der Durchmesser $d_w$ zu $d_n$ von (Dispersität) 1,0 bis 10,0, bevorzugt 1,5 bis 5,0, besonders bevorzugt 2,0 bis 2,6, wobei:

$d_n$ = Zahlenmittelwert des Durchmessers
$d_w$ = Gewichtsmittelwert des Durchmessers

ist.

Die hier benutzten Mittelwerte definieren sich wie folgt:

$$d_n = \Sigma\ n_i\ \mathrm{x}\ d_i\ /\ \Sigma\ n_i \qquad = \text{Zahlenmittelwert}$$

$$d_w = \Sigma\ n_i\ \mathrm{x}\ d_i^{\,2}\ /\ \Sigma\ n_i\ \mathrm{x}\ d_i \qquad = \text{Gewichtsmittelwert}$$

$n_i$ = Anzahl der Partikel mit dem Durchmesser $d_i$,
$d_i$ = ein bestimmter Durchmesser,
i = fortlaufender Parameter.

Der Begriff "Gewicht" steht hier nicht für Masse, sondern für ein gewichtetes Mittel. Die größeren Durchmesser erhalten einen höheren Stellenwert; durch den Exponenten 2 werden Durchmesser größerer Partikel stärker gewichtet.

Die Dispersität der Verteilung der Durchmesser bei den Partikeln ist definiert als:

$$D = d_w/d_n$$

Die Uneinheitlichkeit der Verteilung der Durchmesser ist definiert als:

$$U = d_w/d_n - 1 = D - 1$$

Je näher der Wert für die Uneinheitlichkeit bei "0" liegt, desto einheitlicher sind die Partikel hinsichtlich der Verteilung ihrer Größe geformt.

Die mikrosphärischen Kristallite können besonders auch wegen ihrer einheitlichen Gestalt und Größe in verschiedenen Anwendungsbereichen, entweder als solche in reiner Form oder dadurch, daß Wirksubstanzen im weitesten Sinn eingeschlossen sind, vorteilhaft eingesetzt werden, so z.B.

- als Additive für die Kosmetik in Salben, Pudern, Cremes, Pasten etc.,
- als Träger für Wirksubstanzen in pharmazeutischen und anderen Anwendungen,
- als Glättungsmittel, z.B. zum Verschließen von Poren oder Glätten von Graten,
- als Lebensmittelzusatzstoff, z.B. als Füllkomponente oder zum Verbessern von rheologischen Eigenschaften,
- als Additiv zur Veredelung von z.B. Emulsionspolymerisaten,
- als Trennhilfen, z.B. in der Abtrennung von Verunreinigungen,
- als Verkapselungsmaterial,
- als Träger für magnetische Partikel,
- als Füllmittel für bioabbaubare Polymere oder technische Polymere zur Eigenschaftskontrolle,
- Keimbildungshilfsmittel zur Förderung der Kristallisation oder Hebung des kristallinen Anteiles in synthetischen Massekunststoffen,
- als Additiv zur Eigenschaftskonlrolle, z.B. der Porosität, des Gewichts, der Farbe usw.,
- als Partikelstandard zur Eichung oder Bestimmung der Partikelgröße unbekannter Materialien.

Einzelne Wirksubstanzen oder Wirkstoffkombinationen können z.B. folgender Aufstellung entnommen werden: Pharmazeutische Wirkstoffe, Medikamente, Arzneistoffe, Peptide, Proteine, Nucleinsäuren, Vakzine, Antikörper, Steroide, Oligonucleotide, Aromen, Duftstoffe, Dünger, agritechnische Wirksubstanzen wie Pestizide, Herbizide, Insektizide, Fungizide, Chemikalien mit speziellen Eigenschaften wie Leuchtstoffe, Emulgatoren, Tenside, Pigmente, Oxidationsmittel, Reduktionsmittel, Fullerene, magnetische Komplexe, z.B. paramagnetische Verbindungen.

Somit ist ein weiterer Gegenstand der Erfindung die Verwendung der vorstehend beschriebenen mikrosphärischen Kristallite zur kontrollierten z.B. einer retardierten Abgabe von Wirkstoffen.

Bei dem erfindungsgemäßen Verfahren handelt es sich um eine sehr einfache Vorgehensweise. Die Parameter zur Herstellung der Partikel können in weiten Bereichen wie Verhältnis Lösungsmittel zu Fällungsmittel, Temperatur während des Ausfällprozesses, Konzentration der Lösung, Geschwindigkeit der Zugabe der Lösung zum Fällungsmittel vorgegeben werden.

Die Partikel zeichnen sich durch eine hohe Einheitlichkeit hinsichtlich ihrer Größe und der Verteilung ihrer Durchmesser aus.

Durch die Unlöslichkeit des erfindungsgemäß bevorzugten 1,4-$\alpha$-D-Polyglucan im Wasser < 100 °C lassen sich besonders vorteilhaft Anwendungen verwirklichen, die nicht eine schnelle Zerstörung der mikrosphärischen Kristallite im Wasser zum Zwecke haben, und daher auch besonders vorteilhaft in Produkten verwendet werden können, in denen Wasser als eine weitere Komponente enthalten ist.

Die mikrosphärischen Kristallite zeichnen sich durch die Fähigkeit aus, daß sie einer hohen mechanischen Belastbarkeit ausgesetzt werden können.

Insbesondere wirken die Partikel aufgrund ihrer Morphologie und Einheitlichkeit glättend, z.B. von Poren.

Das bevorzugt zum Einsatz gelangende 1,4-$\alpha$-D-Polyglucan kann auf verschiedene Weisen hergestellt werden. Eine sehr vorteilhafte Methode wird in der WO 95/31 553 beschrieben. Auf die Offenbarung in dieser Schrift wird sich hier ausdrücklich bezogen.

Die folgenden Figuren erläutern die Erfindung näher:

Figur 1 zeigt mikrosphärische Kristallite, hergestellt nach Beispiel 1. Die Kantenlänge des Bilds beträgt 23,28 $\mu$m.

Figur 2 zeigt mikrosphärische Kristallite, hergestellt nach Beispiel 1. Die Kantenlänge des Bilds beträgt 23,3 $\mu$m.

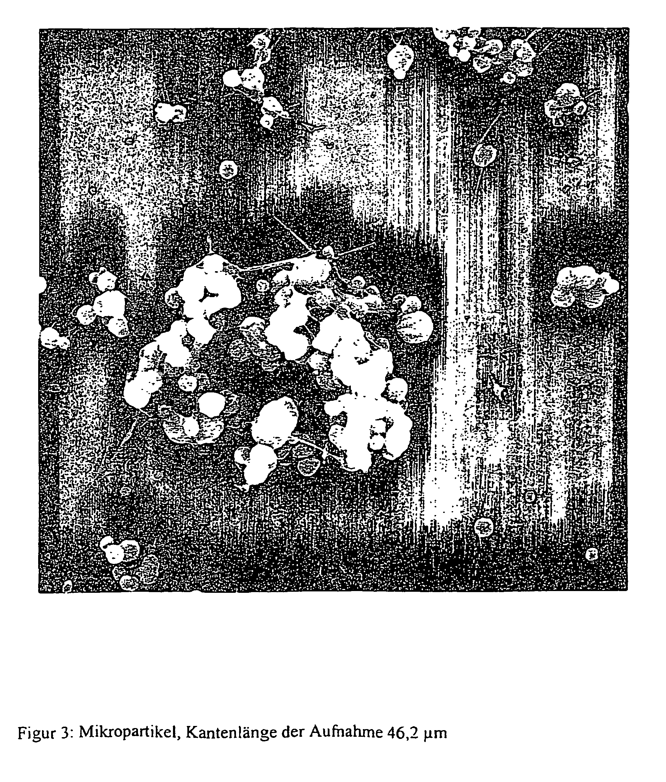

Figur 3 zeigt mikrosphärische Kristallite, hergestellt nach Beispiel 6. Die Kantenlänge des Bilds beträgt 46,2 $\mu$m.

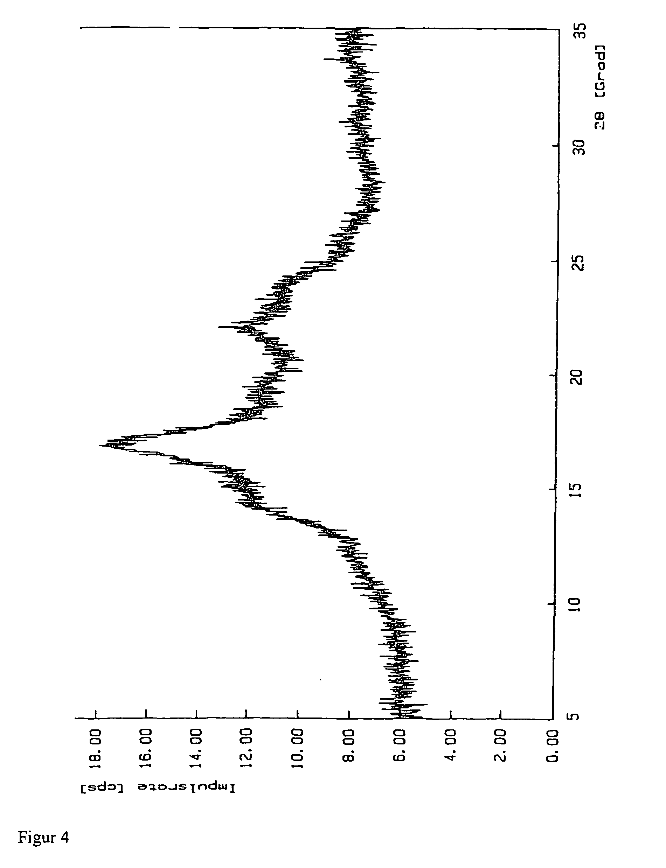

In Figur 4 ist eine Weitwinkelstreuungsbestimmung von 1,4-$\alpha$-D-Polyglucan, A-Typ in Form von erfindungsgemäßen Mikropartikeln gezeigt. Der Kristallinitätsgrad ist >95%.

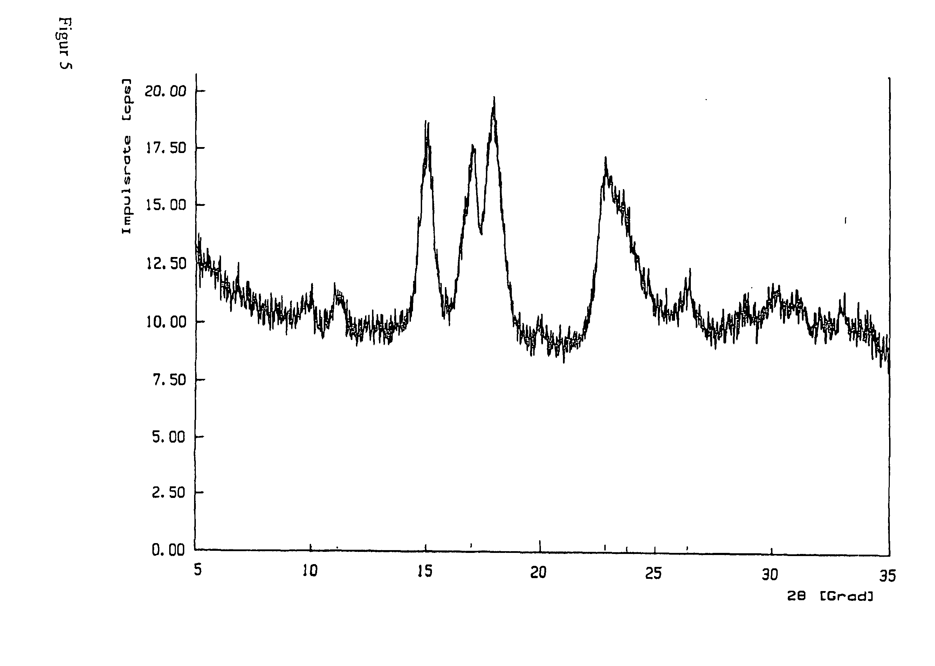

In Figur 5 ist eine solche Weitwinkelstreuungsbestimmung von Neoamylose des B-Typs in nicht mikropartikulärer Form gezeigt. Der Kristallinitätsgrad ist < 50%.

Die Erfindung wird anhand der folgenden Beispiele näher erläutert. Die Beispiele dienen der Veranschaulichung und haben keine limitierende Bedeutung.

**Beispiele:**

**Beispiel 1**

Herstellung von mikrosphärischen Kristalliten aus 1,4-$\alpha$-D-Polyglucan:

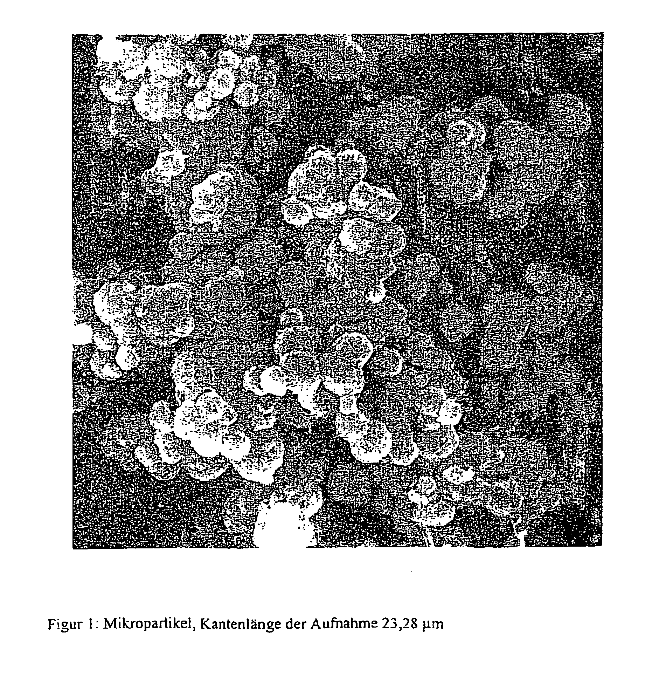

1 g 1,4-$\alpha$-D-Polyglucan, werden in 5 ml Wasser im Autoklaven bei 140°C geschmolzen. Die Schmelze wird auf 80 °C im Autoklaven abgekühlt und bei dieser Temperatur 15 Minuten gelagert. Die Suspension wird im Anschluß gefriergetrocknet. Es werden 850 mg farblose 1,4-a-D-Polyglucan Partikel erhalten. Dies entspricht einer Ausbeute von 85%. Die Charakterisierung der Partikel erfolgte anhand von Rasterelektronenmikroskopaufnahmen (REM, Camscan S-4). Die Partikel sind in Abbildung 1 gezeigt. Die Kantenlänge des Bildes beträgt 23,0 $\mu$m.

**Beispiele 2 und 3:**

Jeweils 100 mg 1,4-$\alpha$-D-Polyglucan und eine den Konzentrationsangaben in Tabelle 1 entsprechende Menge Hydroxypropylmethylcellulose (HPMC, E5Prem., Dow Chemicals) wird in 5 ml Wasser bei 140°C im Autoklaven geschmolzen. Die Schmelze wird auf 90 °C abgekühlt und 15 Minuten bei dieser Temperatur gelagert.

Die erhaltene Suspension wird eingefroren und lyophilisiert (Gefriertrocknung Christ Delta 1-24KD).

Die Ergebnisse sind in der folgenden Tabelle 1 aufgeführt.

**Tabelle 1**

|            | Konzentration HPMC (%) | Ausbeute (%) |
|------------|------------------------|--------------|
| **Beispiel 2** | 1,0                    | 98           |
| **Beispiel 3** | 10,0                   | 89           |

Die Charakterisierung der Partikel erfolgte anhand von Rasterelektronenmikroskopaufnahmen (REM, Camscan S-4).

Die Ergebnisse sind in der folgenden Tabelle 2 aufgeführt.

**Tabelle 2**

|                          | Beispiel 2     | Beispiel 3           |
|--------------------------|----------------|----------------------|
| **Größe**                | 1,0-3,0 $\mu$m | 1,0-3,0 $\mu$m       |
| **Form**                 | kugelförmig    | nahezu kugelförmig   |
| **Oberflächenbeschaffenheit** | glatt     | glatt                |

**Beispiele 4 und 5:**

Einfluß des Molekulargewichts des Cellulosederivats auf die Partikelbeschaffenheit

Die Versuche wurden im wesentlichen analog zu den Beispielen 1 und 2 durchgeführt mit der Ausnahme, daß HPMC unterschiedlicher Molekulargewichte verwendet wurde, wobei E5Prem niedermolekularer Natur und damit niederviskoser als K15Prem (ebenfalls von Dow Chemical) ist.

Wie aus den Ergebnissen ersichtlich ist, sind die erhaltenen Partikel von gleicher Qualität. Das Molekulargewicht des eingesetzten Cellulosederivats hat folglich keinen wesentlichen Einfluß auf die Beschaffenheit der Partikel.

Die Ergebnisse sind in der folgenden Tabelle 3 zusammengefaßt.

**Tabelle 3**

|  | **Beispiel 4** | **Beispiel 5** |
|---|---|---|
| **Konzentration** | E5Prem | K15Prem |
| **HPMC (%)** | 1,0 | 1,0 |
| **Ausbeute %** | 85 | 90 |
| **Größe** | 1,0-3,0 $\mu$m | 1,0-3,0 $\mu$m |
| **Form** | kugelförmig | kugelförmig |
| **Oberflächenbeschaffenheit** | nahezu glatt | nahezu glatt |

**Beispiel 6:**

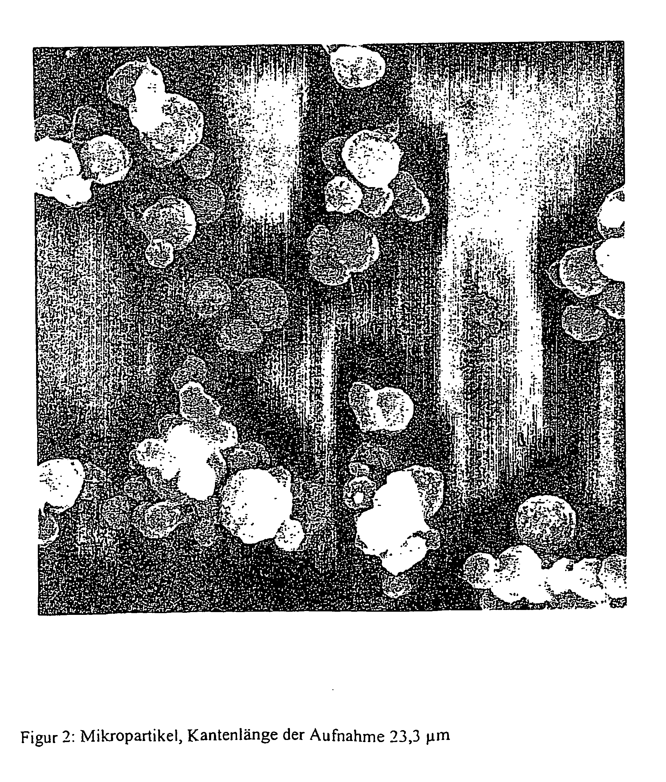

40 g 1,4-a-D-Polyglucan werden in 100 ml Wasser bei 180 °C im Autoklav geschmolzen. Die Schmelze wird im geschlossenen Autoklaven auf 80 °C abgekühlt und 15 Minuten bei dieser Temperatur gelagert. Die erhaltene Suspension wird gefroren und gefriergetrocknet. Die Ausbeute der trockenen mikrosphärischen Kristallite beträgt 90 %. Die mikrosphärischen Kristallite zeigen einen mittleren Durchmesser $d_w$ 100 nm und $d_N$ 90 nm. Die Abbildung 2. wurde im Rasterelektronenmikroskop aufgenommen. Die Kantenlänge des Bildes beträgt 4,65 $\mu$m.

**Patentansprüche**

1. Verfahren zur Herstellung von mikrosphärischen Kristalliten, welche ganz oder teilweise aus linearen und wasserunlöslichen Polysacchariden, bestehen, **dadurch gekennzeichnet, daß** man

   (i) das (die) lineare(n) Polysaccharid(e) in Wasser bei >130°C schmilzt, und durch
   (ii) Fällen in einem Fällungsmittel bei <90°C die mikrosphärischen Kristallite herstellt,

   wobei die Polysaccharide den Löslichkeitsklassen 4 bis 7, bevorzugt den Löslichkeitsklassen 5-7, besonders bevorzugt den Löslichkeitsklassen 6-7 und ganz besonders bevorzugt der Löslichkeitsklasse 7 des DAB entsprechen, und
   wobei linear bedeutet, daß der Verzweigungsgrad maximal 2,5% beträgt.

2. Verfahren nach Anspruch 1, **dadurch gekennzeichnet, daß** man 1,4-$\alpha$-D-Polyglucan, vorgängig zur Bildungen von mikrosphärischen Kristalliten mehrmals im Wasser schmilzt, in einem Fällmittel fällt, den Niederschlag von dem Überstand trennt, den Überstand verwirft und den Niederschlag gegebenenfalls mit Wasser wäscht.

3. Verfahren nach einem der vorstehenden Ansprüche, **dadurch gekennzeichnet, daß** man das Schmelzen bei Temperaturen von 130°C-200°C und das Fällen bei einer Temperatur von 0°C-90°C, bevorzugt bei 20-70°C vornimmt.

4. Verfahren nach einem der vorstehenden Ansprüche, **dadurch gekennzeichnet, daß** man das Schmelzen des Polysaccharids und die Bildung der Mikrokristallite in einem geschlossenen Gefäß beim Überdruck des Wasserdampfes durchführt.

5. Verfahren nach einem der vorstehenden Ansprüche, **dadurch gekennzeichnet, daß** man als Fällmittel Wasser oder ein wäßriges Medium verwendet.

6. Verfahren nach einem der vorstehenden Ansprüche, **dadurch gekennzeichnet, daß** man die Schmelze in Gegenwart eines oder mehrerer Polymere, insbesondere biologisch abbaubarer Polymere und/oder eines oder mehrerer Wirkstoffe herstellt.

7. Verfahren nach einem der vorstehenden Ansprüche, **dadurch gekennzeichnet, daß** das eingesetzte Polysaccharid durch ein fermentatives oder enzymatisches Verfahren hergestellt ist.

8. Verfahren nach einem der vorstehenden Ansprüche, **dadurch gekennzeichnet, daß** man die Schmelze in Gegenwart mindestens eines wasserlöslichen Cellulosederivates herstellt.

**9.** Verfahren nach Anspruch 7, **dadurch gekennzeichnet, daß** das verwendete Enzym ausgewählt ist aus der Gruppe bestehend aus: Polysaccharidsynthase, Stärkesynthase, Glykosyltransferase, a-1,4-Glucantransferase, Glycogensynthase, Amylosucrase und Phosphorylase.

**10.** Verfahren nach einem der vorstehenden Ansprüche, **dadurch gekennzeichnet, daß** das Polysaccharid Poly-1,4-$\alpha$-D-Glucan umfaßt.

**11.** Verfahren nach einem der vorstehenden Ansprüche, **dadurch gekennzeichnet, daß** das Polysaccharid ausschließlich Poly-1,4-$\alpha$-D-Glucan ist.

**12.** Mikrosphärische Kristallite, hergestellt nach einem der vorstehenden Ansprüche.

**13.** Mikrosphärische Kristallite nach Anspruch 12 mit einem mittleren Durchmesser von 1 nm bis 100 $\mu$m, wobei die Partikel eine Dispersität in einem Bereich von 1,0 bis 10,0 aufweisen und separiert vorliegen.

**14.** Mikrosphärische Kristallite nach einem der Ansprüche 12-13, **dadurch gekennzeichnet, daß** sie einen Kristallinitätsgrad von vorzugsweise >80%, besonders bevorzugt >90%, ganz besonders bevorzugt > 95% und am meisten bevorzugt > 98% aufweisen

**15.** Mikrosphärische Kristallite nach einem der Ansprüche 12-14, **dadurch gekennzeichnet, daß** die zu ihrer Herstellung verwendeten linearen Polysacchariden durch enzymatische Behandlung von verzweigten oder hochverzweigten Polysacchariden hergestellt wurden.

**16.** Mikrosphärische Kristallite nach einem der Ansprüche 12-15 mit einem mittleren Durchmesser von 50 nm bis 10 $\mu$m, vorzugsweise 100 nm bis 3 $\mu$m.

**17.** Mikrosphärische Kristallite nach einem der Ansprüche 12-16, **gekennzeichnet durch** eine Dispersität der Partikeldurchmesser $d_w$ zu $d_n$ von 1,5 bis 5,0, insbesondere 2,0 bis 2,6.

**18.** Mikrosphärische Kristallite nach einem der Ansprüche 12-17, **dadurch gekennzeichnet, daß** sie zusätzlich ein oder mehrere, vorzugsweise biologisch abbaubare(s) Polymer(e) enthalten.

**19.** Mikrosphärische Kristallite nach einem der Ansprüche 12-18, **dadurch gekennzeichnet, daß** sie zusätzlich einen oder mehrere Wirkstoffe enthalten.

**20.** Verwendung von mikrosphärischen Kristalliten nach einem der Ansprüche 12-19 in kosmetischen Zubereitungen.

**21.** Verwendung von mikrosphärischen Kristalliten nach einem der Ansprüche 12-20 in Lebensmittelzubereitungen.

**22.** Verwendung von mikrosphärischen Kristalliten nach einem der Ansprüche 12-21 in pharmakologischen Zubereitungen.

**Claims**

**1.** A method for producing microspherical crystallites which consist wholly or partly of linear and water-insoluble polysaccharides, which comprises

(i) melting the linear polysaccharide(s) in water at > 130°C, and
(ii) producing the microspherical crystallites by precipitation in a precipitant at < 90°C,

where the polysaccharides correspond to solubility classes 4 to 7, preferably to solubility classes 5-7, particularly preferably to solubility classes 6-7 and very particularly preferably to solubility class 7 of the DAB, and where linear means that the degree of branching does not exceed 2.5%.

**2.** The method as claimed in claim 1, wherein the 1,4-$\alpha$-D-polyglucan is, previous to the formations of microspherical crystallites, subjected more than once to melting in water, precipitation in a precipitant, separation of the precipitate from the supernatant, discarding of the supernatant, and washing the precipitate where appropriate with water.

3. The method as claimed in either of the preceding claims, wherein the melting is carried out at temperatures of 130°C-200°C and the precipitation is carried out at a temperature of 0°C-90°C, preferably at 20-70°C.

4. The method as claimed in any of the preceding claims, wherein the melting of the polysaccharide and the formation of the microcrystallites are carried out in a closed vessel with the excess pressure of the water vapor.

5. The method as claimed in any of the preceding claims, wherein water or an aqueous medium is used as precipitant.

6. The method as claimed in any of the preceding claims, wherein the melt is produced in the presence of one or more polymers, in particular biodegradable polymers, and/or of one or more active substances.

7. The method as claimed in any of the preceding claims, wherein the polysaccharide employed is produced by a fermentation or enzymatic method.

8. The method as claimed in any of the preceding claims, wherein the melt is produced in the presence of at least one water-soluble cellulose derivative.

9. The method as claimed in claim 7, wherein the enzyme used is selected from the group consisting of: polysaccharide synthase, starch synthase, glycosyltransferase, $\alpha$-1,4-glucan transferase, glycogen synthase, amylosucrase and phosphorylase.

10. The method as claimed in any of the preceding claims, wherein the polysaccharide comprises poly-1,4-$\alpha$-D-glucan.

11. The method as claimed in any of the preceding claims, wherein the polysaccharide is exclusively poly-1,4-$\alpha$-D-glucan.

12. Microspherical crystallites produced as claimed in any of the preceding claims.

13. Microspherical crystallites as claimed in claim 12 with an average diameter of from 1 nm to 100 $\mu$m, the particles having a dispersity in a range from 1.0 to 10.0 and being separate.

14. Microspherical crystallites as claimed in either of claims 12-13, which have a degree of crystallinity preferably of > 80%, particularly preferably of > 90%, very particularly preferably of > 95% and most preferably of > 98%.

15. Microspherical crystallites as claimed in any of claims 12-14, wherein the linear polysaccharides used to produce them have been produced by enzymatic treatment of branched or highly branched polysaccharides.

16. Microspherical crystallites as claimed in any of claims 12-15 with an average diameter of from 50 nm to 10 $\mu$m, preferably 100 nm to 3 $\mu$m.

17. Microspherical crystallites as claimed in any of claims 12-16, having a dispersity of the particle diameters $d_w$ to $d_n$ of from 1.5 to 5.0, in particular 2.0 to 2.6.

18. Microspherical crystallites as claimed in any of claims 12-17, which additionally comprise one or more, preferably biodegradable, polymer(s).

19. Microspherical crystallites as claimed in any of claims 12-18, which additionally comprise one or more active substances.

20. The use of microspherical crystallites as claimed in any of claims 12-19 in cosmetic preparations.

21. The use of microspherical crystallites as claimed in any of claims 12-20 in food preparations.

22. The use of microspherical crystallites as claimed in any of claims 12-21 in pharmacological preparations.

**Revendications**

1. Procédé de production de cristallites microsphériques qui consistent en totalité ou en partie en polysaccharides linéaires et insolubles dans l'eau, **caractérisé en ce que**

    (i) on fait fondre dans l'eau à>130°C le ou les polysaccharides linéaires, et
    (ii) on produit les cristallites microsphériques par précipitation dans un agent de précipitation à <90°C,

    où les polysaccharides correspondent aux classes de solubilité 4 à 7, de préférence aux classes de solubilité 5-7, de manière particulièrement préférée aux classes de solubilité 6-7 et de manière tout particulièrement préférée à la classe de solubilité 7 du DAB, et
    où linéaire signifie que le degré de ramification est au maximum 2,5 %.

2. Procédé selon la revendication 1 **caractérisé en ce que** l'on fait fondre plusieurs fois dans l'eau du 1,4-$\alpha$-D-polyglucane, utilisable pour la formation de cristallites microsphériques, on le précipite dans un agent de précipitation, on sépare le précipité du surnageant, on rejette le surnageant et on lave éventuellement le précipité avec de l'eau.

3. Procédé selon l'une des revendications précédentes **caractérisé en ce que** l'on conduit la fusion à des températures de 130°C 200°C et la précipitation à une température de 0°C-90°C, de préférence à 20-70°C.

4. Procédé selon l'une des revendications précédentes **caractérisé en ce que** l'on conduit la fusion du polysaccharide et la formation des microcristallites dans un récipient fermé sous pression de vapeur d'eau.

5. Procédé selon l'une des revendications précédentes **caractérisé en ce que** l'on utilise l'eau ou un milieu aqueux comme agent de précipitation.

6. Procédé selon l'une des revendications précédentes **caractérisé en ce que** l'on produit la masse fondue en présence d'un ou plusieurs polymères, en particulier de polymères biologiquement dégradables et/ou d'un ou plusieurs principes actifs.

7. Procédé selon l'une des revendications précédentes **caractérisé en ce que** le polysaccharide utilisé est produit par un procédé à fermentation ou enzymatique.

8. Procédé selon l'une des revendications précédentes **caractérisé en ce que** l'on produit, la masse fondue en présence d'au moins un dérivé cellulosique soluble dans l'eau.

9. Procédé selon la revendication 7 **caractérisé en ce que** l'enzyme utilisée est choisie dans le groupe consistant en la polysaccharide synthase, l'amidon synthase, la glycosyltransférase, l'$\alpha$-1,4-glucanetransférase, la glycogènesynthase, l'amylosaccharase et la phosphorylase.

10. Procédé selon l'une des revendications précédentes **caractérisé en ce que** le polysaccharide est le poly-1,4-$\alpha$-D-glucane.

11. Procédé selon l'une des revendications précédentes **caractérisé en ce que** le polysaccharide est exclusivement le poly-1,4-$\alpha$-D-glucane.

12. Cristallites microsphériques produits selon l'une des revendications précédentes.

13. Cristallites microsphériques selon la revendication 12 ayant un diamètre moyen de 1 nm à 100 $\mu$m, où les particules présentent une dispersité dans un domaine de 1,0,à 10,0 et sont présentés sous forme séparée.

14. Cristallites microsphériques selon l'une des revendications 12-13 **caractérisés en ce qu'**ils présentent un degré de cristallinité de préférence > 80 %, de manière particulièrement préférée > 90 %, de manière tout particulièrement préférée > 95% et de préférence encore > 98%.

15. Cristallites microsphériques selon l'une des revendications 12-14 **caractérisés en ce que** les polysaccharides linéaires utilisés pour leur production ont été produits par traitement enzymatique de polysaccharides ramifiés ou hautement ramifiés.

**16.** Cristallites microsphériques selon l'une des revendications 12-15 ayant un diamètre moyen de 50 nm à 10 $\mu$m, de préférence de 100 nm à 3 $\mu$m.

**17.** Cristallites microsphériques selon l'une des revendications 12-16 **caractérisés par** une dispersité des diamètres de particules $d_w$ à $d_n$ de 1,5 à 5,0, en particulier de 2,0 à 2,6.

**18.** Cristallites microsphériques selon l'une des revendications 12-17 **caractérisés en ce qu'**ils contiennent en outre un ou plusieurs polymères de préférence biologiquement dégradables.

**19.** Cristallites microsphériques selon l'une des revendications, 12-18 **caractérisés en ce qu'**ils contiennent en outre un ou plusieurs principes actifs. ,

**20.** Utilisation de cristallites microsphériques selon l'une des revendications 12-19 dans des préparations cosmétiques.

**21.** Utilisation de cristallites microsphériques selon l'une des revendications 12-20 dans des préparations alimentaires.

**22.** Utilisation de cristallites microsphériques selon l'une des revendications 12-21 dans des préparations pharmacologiques.

Figur 1: Mikropartikel, Kantenlänge der Aufnahme 23,28 µm

Figur 2: Mikropartikel, Kantenlänge der Aufnahme 23,3 μm

Figur 3: Mikropartikel, Kantenlänge der Aufnahme 46,2 µm

Figur 4

Figur 5